# FASCICULE DE BREVET EUROPEEN

(11) **EP 4 072 637 B1**
(45) Date de publication et mention de la délivrance du brevet: **10.04.2024**
(21) Numéro de dépôt: 20820414.9
(22) Date de dépôt: 09.12.2020
(51) Int. Cl.: A61M 16/00, A61M 16/16

(54) **DISPOSITIF D'HUMIDIFICATION POUR APPAREIL RESPIRATOIRE À PRESSION POSITIVE**
BEFEUCHTUNGSVORRICHTUNG FÜR EIN ATEMGERÄT MIT POSITIVEM ATEMWEGSDRUCK
HUMIDIFICATION DEVICE FOR A POSITIVE AIRWAY PRESSURE BREATHING APPARATUS

(30) Priorité: 13.12.2019 FR 1914406
(43) Date de publication de la demande: 19.10.2022
(73) Titulaire: Sleepinnov Technology, 38430 Moirans (FR)
(72) Inventeur: HUNGR, Nikolai, 38430 MOIRANS (FR); ARGOD, Jérôme, 38430 MOIRANS (FR)
(74) Mandataire: INNOV-GROUP
(86) Numéro de dépôt international: PCT/EP2020/085390
(87) Numéro de publication internationale: WO 2021/116235

(56) Documents cités:
- WO-A1-2015/103904
- WO-A1-2019/111110
- CA-A1- 2 859 994
- FR-A1- 3 081 713
- US-B2- 9 010 324

## Description

### DOMAINE TECHNIQUE

Le domaine technique de l'invention est un dispositif de ventilation par pression positive continue. Ce type de dispositif est couramment utilisé dans le traitement de l'apnée du sommeil.

### ART ANTERIEUR

Le recours à la ventilation par pression positive continue (PPC) constitue un traitement de référence dans le domaine de l'apnée du sommeil. Ce traitement consiste à insuffler de l'air de façon continue dans un masque appliqué sur le visage d'un utilisateur. Il peut s'agir d'un masque nasal, narinaire ou facial. Le souffle d'air parvient aux voies respiratoires de l'utilisateur, en exerçant une pression suffisante sur ces dernières, de façon à prévenir la formation d'un collapsus. L'air insufflé est généré par un dispositif respiratoire à pression positive continue, ce type d'appareil disposant d'un ventilateur pour générer un écoulement d'air en direction du masque.

Afin d'améliorer le confort d'utilisation, certains dispositifs respiratoires à pression positive continue peuvent comporter un humidificateur. L'humidificateur comporte une réserve d'eau. Il est configuré de telle sorte que l'air circule en dessus de la réserve d'eau avant d'être envoyé dans un conduit débouchant sur le masque.

Le document EP2703034 décrit un dispositif de ventilation pouvant comporter un humidificateur. Il en est de même du document EP2540335 ou du document FR3068610.

Dans le dispositif décrit dans US9010324, une chambre de ventilation est superposée à un humidificateur. Dans ce dispositif, l'humidificateur présente une forme complexe, et n'est pas aisément accessible. Cela rend le nettoyage ou le remplissage peu pratique. De plus, lors de l'utilisation, le circuit électronique se situe en dessous de l'humidificateur, ce qui n'est pas optimal du point de vue de la sécurité d'utilisation. Les inventeurs ont conçu un dispositif de ventilation positive par PPC, en prenant en compte les objectifs suivants :
- disposer d'un dispositif compact ;
- limiter le bruit causé par l'écoulement d'air dans le dispositif ;
- obtenir une bonne sécurité d'utilisation, y compris en cas de renversement du dispositif;
- optimiser le coût et la simplicité de fabrication ;
- faciliter le remplissage et le nettoyage ;
- proposer un dispositif dont la forme autorise d'autres usages.

Ils ont conçu le dispositif décrit ci-après, qui répond à ces objectifs.

### EXPOSE DE L'INVENTION

L'invention est définie par le jeu de revendications.

Un objet de la divulgation est un dispositif de ventilation respiratoire, destiné à envoyer un flux d'air, généré par un ventilateur, dans un conduit, le conduit s'étendant entre le dispositif et un masque respiratoire, destiné à être porté par un utilisateur, le dispositif s'étendant entre une extrémité inférieure, formant un fond, et une extrémité supérieure, le dispositif comportant:
- une chambre de ventilation, renfermant le ventilateur ;
- un humidificateur, relié à la chambre de ventilation, et destiné à humidifier de l'air insufflé par le ventilateur, l'humidificateur comportant un réservoir destiné à recevoir de l'eau ; l'humidificateur comportant :
   - une chambre de liaison, adjacente de la chambre de ventilation, et comprenant :
      - une ouverture d'admission, débouchant de la chambre de ventilation ;
      - une ouverture d'évacuation, débouchant sur canal d'évacuation, le canal d'évacuation étant configuré pour être raccordé au conduit ;
   - une chambre d'humidification, comportant le réservoir ;
   - une première ouverture et une deuxième ouverture, distinctes l'une de l'autre formées entre la chambre de liaison et la chambre d'humidification.

Le dispositif peut comporter au moins l'une des caractéristiques suivantes, prises isolément ou selon les combinaisons techniquement réalisables.
- la chambre de liaison s'étend entre la chambre d'humidification et la chambre de ventilation ;
- le dispositif comporte une paroi de séparation s'étendant entre la chambre de liaison et la chambre d'humidification, la première ouverture et la deuxième ouverture étant ménagées dans la paroi de séparation, la paroi de séparation étant agencée de telle sorte que :
   - la chambre d'humidification s'étend entre le fond et la paroi de séparation;
   - la chambre de liaison s'étend entre la paroi de séparation et la chambre de ventilation.

Le dispositif peut comporter une paroi latérale annulaire, reliant l'extrémité inférieure à l'extrémité supérieure, de telle sorte que la chambre de liaison, la chambre d'humidification et la chambre de ventilation sont délimitées par la paroi latérale annulaire.

La chambre de liaison peut définir un premier canal entre l'ouverture d'admission et la première ouverture, ainsi qu'un deuxième canal entre la deuxième ouverture et l'ouverture d'évacuation, de telle sorte que :
- le premier canal est agencé pour diriger de l'air, provenant la chambre de ventilation, à travers l'ouverture d'admission jusqu'à la chambre d'humidification, à travers la première ouverture;
- le deuxième canal est agencé pour diriger de l'air, provenant de la chambre d'humidification, à travers la deuxième ouverture, jusqu'à la l'ouverture d'évacuation.

Le deuxième canal peut définir un cheminement à travers la chambre de liaison, le cheminement comportant plusieurs coudes entre la deuxième ouverture et l'ouverture d'évacuation.

Selon un mode de réalisation :
- le premier canal s'étend entre deux premières parois transversales, chaque première paroi transversale s'étendant entre la paroi de séparation et une paroi supérieure de la chambre de liaison ;
- et/ou le deuxième canal s'étend entre deux deuxièmes parois transversales chaque deuxième paroi transversale s'étendant entre la paroi de séparation et la paroi supérieure de la chambre de liaison.
- la chambre de liaison s'étend entre la paroi de séparation et la paroi supérieure de la chambre de liaison;
- chaque première paroi transversale et/ou chaque deuxième paroi transversale s'étend à partir de la paroi supérieure de la chambre de liaison.

Selon un mode de réalisation, une ouverture, choisie parmi l'ouverture d'admission ou la première ouverture, est associée à un obturateur, l'obturateur étant configuré pour passer d'une position ouverte, selon laquelle l'ouverture est ouverte, et une position fermée, selon laquelle l'ouverture est fermée, lorsque le dispositif s'incline, au-delà d'une inclinaison critique, par rapport à une position de référence du dispositif. L'obturateur peut être apte à commuter, par gravité, entre la position ouverte et la position fermée.

Selon un mode de réalisation, le dispositif comporte un inclinomètre, configuré pour mesuré un angle d'inclinaison du dispositif par rapport à une position de référence du dispositif, l'inclinomètre étant relié à un coupe-circuit, l'inclinomètre étant configuré pour activer le coupe-circuit, de façon à interrompre un fonctionnement du ventilateur lorsque le dispositif s'incline, au-delà d'une inclinaison critique, par rapport à une position de référence du dispositif.

Selon un mode de réalisation, la paroi latérale annulaire s'étend autour d'un axe transversal, s'étendant entre le fond et l'extrémité supérieure. La paroi annulaire peut être cylindrique ou tronc-conique.

La position de référence du dispositif peut être telle que l'axe transversal est vertical, ou vertical à une tolérance angulaire près, par exemple ±25° ou ±45° ou ±60°.

Selon un mode de réalisation, le réservoir d'eau s'étend, dans la chambre d'humidification, entre le fond du dispositif et la paroi de séparation. Le réservoir d'eau peut être amovible, de telle sorte qu'il peut être extrait de la chambre d'humidification ou inséré dans cette dernière. Le réservoir d'eau est de préférence extrait ou inséré dans la chambre d'humidification par translation parallèlement au fond.

Selon un mode de réalisation, l'ouverture d'évacuation débouche sur un canal d'évacuation coudé.

De préférence, l'humidificateur est amovible, de façon à pouvoir être séparé de la chambre de ventilation du dispositif.

L'humidificateur peut former un socle du dispositif, la chambre de ventilation étant disposée sur l'humidificateur.

Selon un mode de réalisation,
- la chambre de liaison est délimitée par une paroi supérieure, la paroi supérieure étant adjacente de la chambre de ventilation;
- la paroi supérieure est amovible.

L'invention sera mieux comprise à la lecture de l'exposé des exemples de réalisation présentés, dans la suite de la description, en lien avec les figures listées ci-dessous.

### FIGURES

La figure 1A représente un exemple d'utilisation du dispositif.
La figure 1B montre une vue d'ensemble du dispositif.
La figure 1C représente un humidificateur faisant partie du dispositif.
La figure 1D est une coupe, selon un plan YZ, de l'humidificateur.
La figure 2A montre les principaux composants d'une chambre de liaison, faisant partie de l'humidificateur.
La figure 2B est une vue de dessus, selon un plan XY, de la chambre de liaison.
La figure 2C représente la paroi supérieure de la chambre de liaison, cette dernière s'étendant entre la paroi de séparation et la chambre de ventilation.
La figure 2D représente la paroi de séparation, séparant la chambre de liaison de la chambre d'humidification.
La figure 2E montre la chambre d'humidification, ainsi qu'un obturateur associé à une ouverture d'admission, et disposé dans la chambre de liaison.
La figure 3A est une vue générale de la chambre d'humidification.
La figure 3B montre un réservoir de l'humidificateur, destiné à contenir de l'eau.
La figure 3C illustre la disposition du réservoir par rapport à un châssis, dans la chambre d'humidification.
La figure 4 représente un canal d'évacuation, s'étendant entre la chambre de liaison et une sortie du dispositif.
La figure 5 illustre un mode de réalisation du dispositif.

### EXPOSE DE MODES DE REALISATION PARTICULIERS

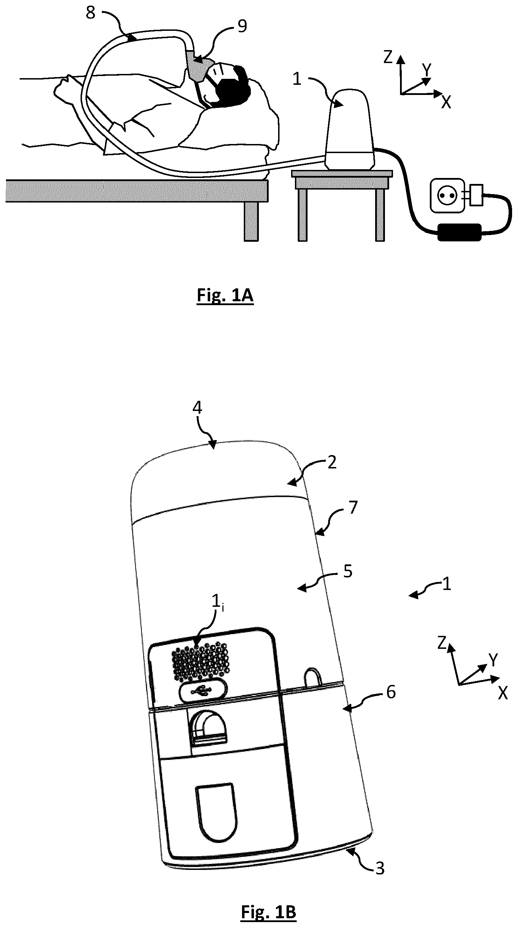

La figure 1A montre un dispositif 1 d'aide à la respiration selon l'invention. Le dispositif comporte un conduit 8, le reliant à un masque respiratoire 9 destiné à être appliqué sur le visage d'un utilisateur. De préférence, le conduit 8 est un conduit souple, dont la longueur est de quelques mètres. Le dispositif 1 comporte une chambre de ventilation 5, renfermant un ventilateur. Ce dernier est commandé pour maintenir une pression de consigne au niveau du masque respiratoire. Il est essentiellement destiné à un usage nocturne. Lors de son utilisation, le dispositif peut notamment être disposé sur un support plan, par exemple une table de chevet.

La figure 1B est une vue d'ensemble du dispositif. Le dispositif s'étend, selon un axe transversal Z, entre une extrémité inférieure 3, formant un fond, et une extrémité supérieure 4. La distance entre le fond 3 et l'extrémité supérieure 4 peut être comprise entre 10 cm et 40 cm. Elle est par exemple égale à 30 cm. Le fond 3 s'étend de préférence selon un plan de base P_{XY}, défini par des axes X et Y. Le fond peut par exemple être circulaire. Selon le plan de base XY, le fond est inscrit dans un diamètre de préférence inférieur à 15 cm ou 30 cm. Les dimensions du dispositif le rendent aisément transportable. Par ailleurs, le dispositif est adapté à être déposé sur un support plan de faibles dimensions, par exemple une table. Lors de l'utilisation, l'axe transversal Z est de préférence parallèle à la verticale, tandis que le plan de base XY est horizontal.

Dans l'exemple représenté, le dispositif 1 est confiné dans une enceinte 2. Cette dernière est de préférence constituée d'un matériau rigide, par exemple un plastique. Plus précisément, entre le fond 3 et l'extrémité supérieure 4, le dispositif est délimité radialement par une paroi latérale 7, s'étendant autour de l'axe transversal Z. Le dispositif comporte une entrée d'air 1ᵢ ainsi qu'une sortie d'air 1ₒ. L'air admis à travers l'entrée d'air 1ᵢ est dirigé vers le ventilateur de la chambre de ventilation 5, ce dernier générant un flux d'air circulant dans le dispositif 1, jusqu'à une sortie d'air 1ₒ, cette dernière étant raccordée au conduit 8.

Le dispositif peut comporter un capteur de pression, mesurant la pression de l'air entre le ventilateur et la sortie d'air 1ₒ. En fonction de la pression mesurée, une unité de commande adapte la puissance du ventilateur de façon à maintenir une pression aussi stable que possible autour d'une pression de consigne.

La chambre de ventilation 5 est superposée à un humidificateur 6. Selon le sens de l'écoulement de l'air, l'humidificateur est disposé en dessous de la chambre de ventilation. L'humidificateur 6 forme ainsi un socle du dispositif 1. Ainsi, la chambre de ventilation 5 et l'humidificateur 6 sont superposés. L'humidificateur 6 peut être amovible de façon à pouvoir être séparé de la chambre de ventilation 5, lorsque le dispositif n'est pas utilisé. Cela peut permettre de disposer d'un dispositif particulièrement compact, notamment lors de son transport.

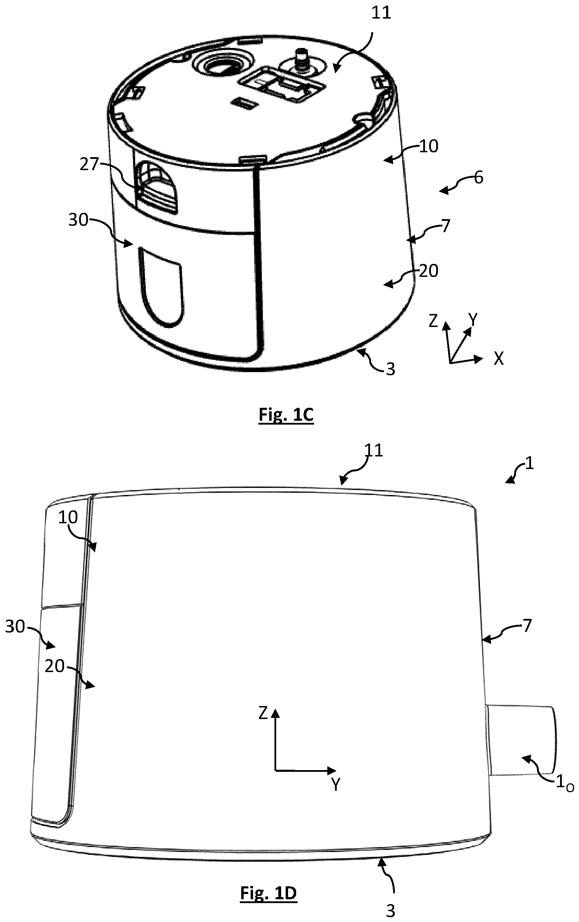

Les figures 1C et 1D représentent l'humidificateur 6. Ce dernier est configuré pour être fixé en dessous de la chambre de ventilation 5. L'humidificateur 6 comporte une chambre de liaison 10 superposée à une chambre d'humidification 20. La chambre d'humidification 20 comporte un réservoir d'eau amovible, que l'on peut retirer ou insérer dans le dispositif par l'intermédiaire d'un volet 30, ménagé dans la continuité de la paroi latérale 7.

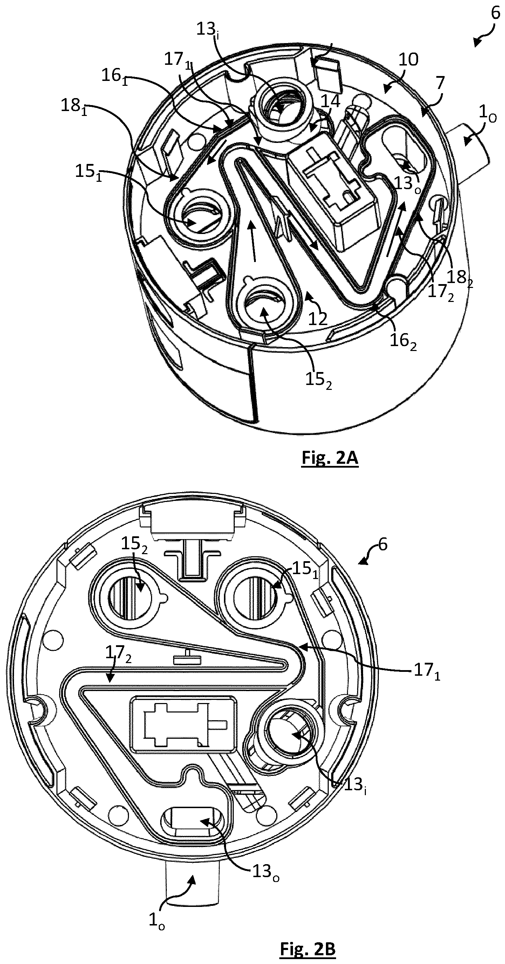

La figure 2A détaille la structure de la chambre de liaison 10. Cette dernière forme une interface entre la chambre de ventilation 5 et la chambre d'humidification 20. Elle forme également une interface entre la chambre d'humidification 20 et la sortie 1ₒ du dispositif. La chambre de liaison 10 s'étend, selon l'axe Z, entre une paroi de séparation 12 et une paroi supérieure 11. Entre la paroi de séparation 12 et la paroi supérieure 11, la chambre de liaison 10 est délimitée par la paroi latérale 7. Ainsi, la paroi latérale 7 relie la paroi de séparation 12 à la paroi supérieure 11. La paroi supérieure 11 effectue une séparation entre la chambre de ventilation 5 et la chambre de liaison 10.

La paroi de séparation 12 effectue une séparation entre la chambre de liaison 10 et la chambre d'humidification 20. La chambre d'humidification 20 s'étend, selon l'axe Z, entre le fond 3 et la paroi de séparation 12. Entre le fond 3 et la paroi de séparation 12, la chambre d'humidification 10 est délimitée par la paroi latérale 7. Ainsi, la paroi latérale 7 relie le fond à la paroi de séparation 12. Dans l'exemple représenté, la paroi de séparation 12 est parallèle au fond 3. C'est également le cas de la paroi supérieure 11.

Une ouverture d'admission 13ᵢ est ménagée à travers la paroi supérieure 11, de façon à permettre à l'air, s'écoulant depuis la chambre de ventilation 5, d'entrer dans la chambre de liaison 10.

La paroi de séparation 12 comporte une première ouverture 15₁ et une deuxième ouverture 15₂, la première ouverture et la deuxième ouverture permettant à l'air de passer de part et d'autre de la paroi de séparation 12, de la chambre de liaison 10 vers la chambre d'humidification 20 et réciproquement

La chambre de liaison 10 comporte un premier canal 16₁, s'étendant de l'ouverture d'admission 13ᵢ à la première ouverture 15₁. Ainsi, durant le fonctionnement du dispositif 1, l'air pénètre dans l'humidificateur 6 par l'ouverture d'admission 13ᵢ, puis s'écoule le long du premier canal 16₁ jusqu'à la première ouverture 15₁. Le premier canal 16₁ est délimité par la paroi de séparation 12 et la paroi supérieure 11. Il est également délimité par une première paroi latérale 17₁ ou des premières parois latérales 17₁, s'étendant entre la paroi de séparation 12 et la paroi supérieure 11. Dans l'exemple représenté, chaque première paroi latérale 17₁ est insérée sur un joint 18₁ formant un contour fermé autour de l'ouverture d'admission 13ᵢ et la première ouverture 15₁. Selon le plan de base XY du dispositif, c'est-à-dire perpendiculairement à l'axe transversal Z, la section du premier canal 16₁ n'est pas uniforme. Ainsi, le premier canal 16₁ comporte un rétrécissement entre l'ouverture d'admission 13ᵢ et la première ouverture 15₁. Cela permet de réduire le bruit généré par l'écoulement d'air.

Le rétrécissement du premier canal 16₁ permet l'agencement d'un coude d'un deuxième canal 16₂ décrit par la suite.

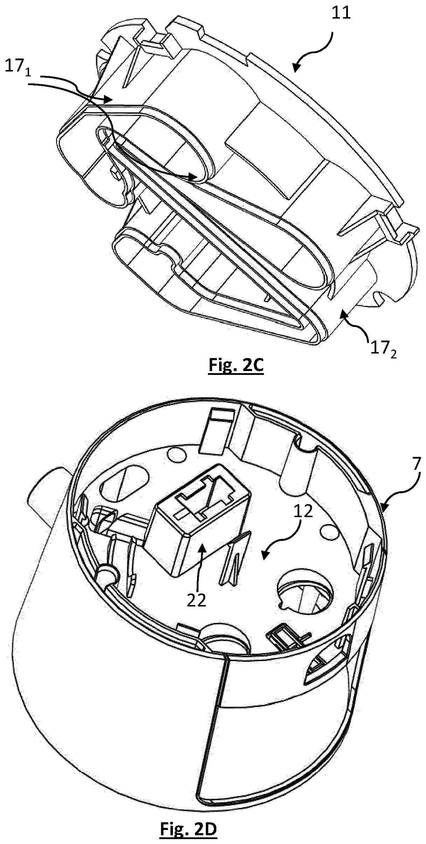

De préférence, chaque paroi latérale 17₁ est solidaire de la paroi supérieure 11, comme représenté sur la figure 2C. Cela simplifie la fabrication du dispositif.

La chambre de liaison 10 comporte un deuxième canal 16₂, s'étendant entre la deuxième ouverture 15₂ et l'ouverture d'évacuation 13ₒ. Ainsi, durant le fonctionnement du dispositif 1, l'air sort de la chambre d'humidification 20 par la deuxième ouverture 15₂, puis s'écoule le long du deuxième canal 16₂ jusqu'à l'ouverture d'évacuation 13ₒ. Le deuxième canal 16₂ est délimité par la paroi de séparation 12 et la paroi supérieure 11. Il est également délimité par une deuxième paroi latérale 17₂ ou des deuxièmes parois latérales 17₂, s'étendant entre la paroi de séparation 12 et la paroi supérieure 11. Dans l'exemple représenté, chaque deuxième paroi latérale 17₂ est insérée sur un joint 18₂ formant un contour fermé autour de l'ouverture d'évacuation 13ₒ et la deuxième ouverture 15₂. De préférence, chaque deuxième paroi latérale 17₂ est solidaire de la paroi supérieure 11, comme représenté sur la figure 2C.

De préférence, la paroi supérieure 11 est amovible, de façon à pouvoir être aisément retirée. Cela permet un accès à l'intérieur de la chambre de liaison 10. Cela facilite un nettoyage de la chambre de liaison, ce qui améliore l'hygiène lors de l'utilisation du dispositif. Cela facilite également la maintenance du dispositif. Dans l'exemple représenté, les parois latérales délimitant le premier canal et le deuxième canal sont solidaires de la paroi supérieure 11 : elles forment alors, avec cette dernière, une même pièce monobloc, comme représenté sur la figure 2C.

Comme on peut le voir sur les figures 2A à 2C, le deuxième canal 16₂ comporte au moins un coude, et de préférence plusieurs coudes entre l'ouverture d'évacuation 13ₒ et la deuxième ouverture 15₂. De préférence, au moins un coude, et de préférence chaque coude, définit un angle supérieur à 90°.

La présence de coudes dans le deuxième canal 16₂ permet d'éviter que de l'eau soit admise dans le conduit 8, en aval de la sortie 1ₒ du dispositif, en cas de mauvaise utilisation ou renversement accidentel du dispositif. Les coudes forment des chicanes, qui gênent un éventuel écoulement d'eau vers l'ouverture d'évacuation 13ₒ.

La figure 2D montre la paroi de séparation 12. La paroi de séparation 12 comporte un renfoncement 22, s'étendant vers l'intérieur de la chambre de liaison 10. Ce renfoncement forme un volume libre pour disposer un connecteur électrique permettant une connexion électrique de composants équipant l'humidificateur. Il peut s'agir d'une plaque chauffante, décrite par la suite, faisant partie de la chambre d'humidification. Il peut également s'agir d'un inclinomètre, décrit par la suite, configuré pour stopper le ventilateur en cas d'inclinaison excessive du dispositif.

Dans l'exemple représenté, l'ouverture d'admission 13ᵢ est associée à un obturateur 14, configuré pour passer d'une position ouverte, autorisant le passage de l'air à travers l'ouverture d'admission, à une position fermée, bloquant le passage de l'air. L'obturateur est configuré pour passer de la position ouverte à la position fermée lorsque le dispositif 1 s'écarte d'une position de référence, correspondant à une utilisation normale du dispositif. La position de référence correspond à la position représentée sur la figure 1A, à savoir le fond 3 disposé en dessous de l'extrémité supérieure 4, l'axe transversal Z étant vertical, à une tolérance angulaire près. La tolérance angulaire peut être par exemple de +/- 25° ou +/- 45°, voire +/- 60°.

Lorsque le dispositif s'écarte de sa position de référence, par exemple en cas de renversement, l'obturateur commute selon la position fermée, ce qui bloque le passage de tout fluide à travers l'ouverture d'admission 13ᵢ. Cela permet d'éviter que de l'eau, contenue dans le réservoir de l'humidificateur, s'écoule, à travers l'ouverture d'admission 13ᵢ, dans la chambre de ventilation 5. On évite alors une dégradation de la chambre de ventilation 5, et par exemple du ventilateur, due à la présence d'eau. On évite également d'éventuels problèmes électriques, la chambre de ventilation comportant des composants électroniques, par exemple au niveau d'une carte de commande du ventilateur.

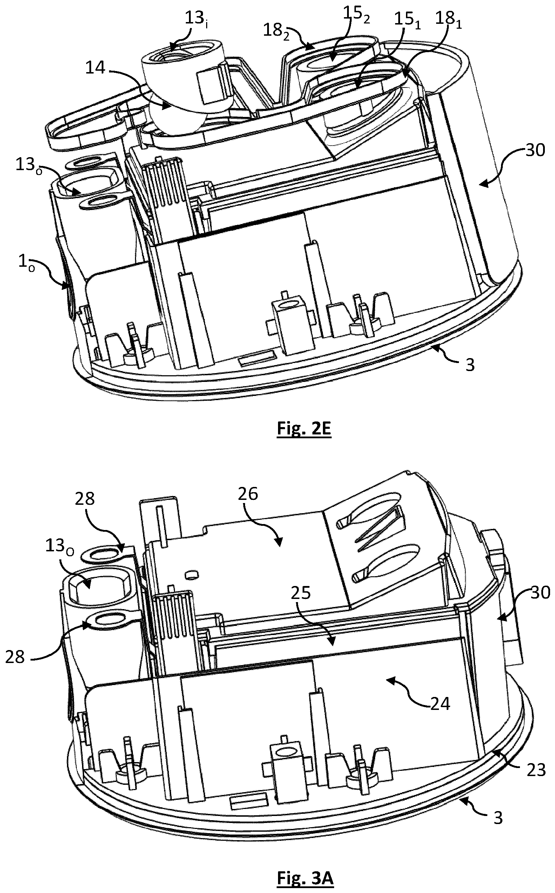

Dans l'exemple représenté, l'obturateur 14 prend la forme d'une bille, reposant sur un support lorsque le dispositif est maintenu selon la position de référence. L'obturateur 14 est visible sur les figures 2A et 2E. En cas d'inclinaison excessive, sous l'effet de la gravité, la bille 14 glisse contre l'ouverture d'admission 13ᵢ de façon à la bloquer. L'obturateur est alors selon la position fermée. En variante, un inclinomètre peut être disposé dans le dispositif 1, de façon à mesurer une inclinaison du dispositif par rapport à la position de référence. En fonction de l'inclinaison du dispositif, un obturateur, de type électrovanne ou clapet, est commandé électroniquement de façon à basculer entre la position ouverte et la position fermée. Dans tous les cas de figure, lorsque le dispositif retrouve une position de référence, l'obturateur commute de la position fermée vers la position ouverte.

En complément ou en remplacement d'un obturateur simple, et actionné par la gravité, le dispositif peut comporter un inclinomètre de façon à générer une alarme sonore et/ou visuelle à destination de l'utilisateur. Cela permet un réveil de l'utilisateur, avant que ce dernier ne perçoive une sensation de manque d'air du fait du passage en position fermée de l'obturateur. Selon un mode de réalisation, lorsque l'inclinaison du dispositif est excessive, le dispositif comporte un coupe circuit de façon à stopper le ventilateur. L'inclinomètre peut fonctionner en utilisant la gravité, ou comporter un capteur de mouvement, par exemple un accéléromètre.

L'obturateur 14 peut également être disposé au niveau de la première ouverture 15₁ au lieu de l'ouverture d'admission 13ᵢ. Un obturateur peut également être disposé au niveau de la deuxième ouverture 15₂, ou de l'ouverture d'évacuation 13ₒ, de façon à éviter un écoulement d'eau dans le conduit 8.

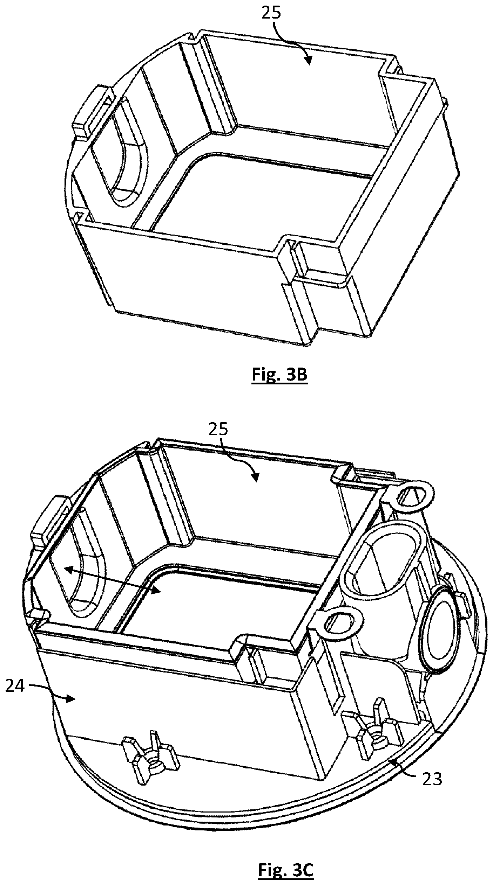

Les figures 3A à 3C concernent la chambre d'humidification 20. Cette dernière comporte un réservoir 25, reposant sur un châssis 24. Le réservoir 25 est configuré pour contenir de l'eau. Durant l'utilisation du dispositif, l'air pénètre dans la chambre d'humidification 20 à travers la première ouverture 15₁ et en ressort à travers la deuxième ouverture 15₂. Au cours de son écoulement dans la chambre d'humidification 20, l'air s'écoule à travers le réservoir 25, et en dessus de l'eau qu'il contient. Il est ainsi humidifié, avant de sortir de la chambre d'humidification 20 et d'être dirigé, à travers la chambre de liaison 10, vers l'ouverture d'évacuation 13₀ puis la sortie 1ₒ du dispositif. Le réservoir peut être disposé sous un couvercle 26, ce dernier comportant des ouvertures, par exemple ménagées en vis-à-vis de la première ouverture et la deuxième ouverture, et autorisant le passage de l'air.

La chambre d'humidification 20 peut comporter une plaque chauffante 23, configurée pour chauffer l'eau du réservoir 25. La température de chauffe est par exemple comprise entre 40°C et 80°C. De préférence, la plaque chauffante 23 est montée sur un ressort, de façon à être plaquée contre le réservoir 25.

Le réservoir 25 est de préférence amovible par rapport au châssis 24. Il peut en particulier être mobile en translation par rapport au châssis 24, de façon à pouvoir être retiré de la chambre d'humidification 20 ou inséré dans cette dernière. Un mécanisme à ressort 28 facilite le retrait du réservoir. Les ressorts sont armés lorsque le réservoir est introduit dans le châssis 24. Sous l'effet d'une pression exercée sur un curseur 27, représenté sur la figure 1C, les ressorts se détendent et facilitent une translation du réservoir 25 par rapport au châssis 24. La translation du réservoir par rapport au châssis s'effectue selon le plan de base XY, c'est-à-dire orthogonalement à l'axe transversal Z. Sur la figure 3C, la double flèche symbolise la direction de translation. Le réservoir est relié au volet 30 représenté sur les figures 1C et 1D. Le volet 30 fait partie de la paroi latérale 7. Il permet une préhension du réservoir 25. Le fait que le réservoir 25 soit amovible, par une simple translation, favorise le remplissage de l'eau. Il permet également un nettoyage du réservoir, ce qui rend l'utilisation du dispositif plus hygiénique. Il en résulte un dispositif particulièrement simple à utiliser et à nettoyer.

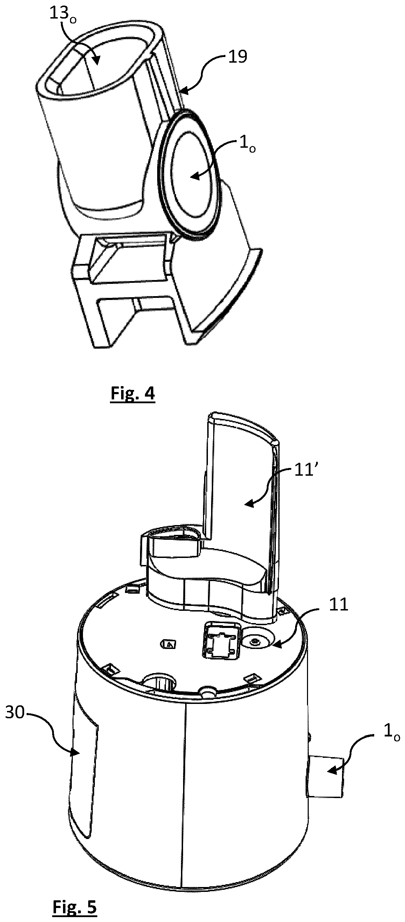

Sur la figure 4, on a représenté le canal d'évacuation 19, s'étendant entre l'ouverture d'évacuation 13ₒ et la sortie 1ₒ du dispositif. Le canal d'évacuation 19 est coudé, ce qui contribue à réduire le bruit d'écoulement d'air. Le canal d'évacuation 19 peut être réalisé, par exemple par moulage, dans un matériau souple, par exemple un élastomètre. Cela contribue également à limiter le bruit d'écoulement d'air. A l'opposé de l'ouverture d'évacuation 13ₒ, le canal d'évacuation 19 débouche sur la sortie 1ₒ du dispositif. Le canal d'évacuation 19 est de préférence amovible, ce qui facilite le nettoyage du dispositif.

Sur la figure 5, on a représenté un mode de réalisation, dans lequel le dispositif comporte un réservoir tampon 11', formant un volume libre. Le réservoir tampon est formé dans la paroi supérieure 11. Il est ouvert sur la chambre de liaison 10. Il est disposé entre la chambre de liaison 10 et la chambre de ventilation 5. En cas de renversement du dispositif, ou d'inclinaison excessive, le réservoir tampon 11' est agencé pour recevoir l'eau se déversant à partir du réservoir 25, de façon à éviter que l'eau pénètre dans la chambre de ventilation. Le réservoir tampon 11' est avantageusement disposé face au premier canal 16₁, par exemple face à l'ouverture d'admission 13ᵢ ou face à la première ouverture 15₁.

Lorsque le dispositif fonctionne, l'humidificateur 6 forme un socle à la chambre de ventilation 5. Cette dernière comporte une alimentation électrique ainsi que des cartes électronique de commande. La chambre de ventilation 5 peut être telle que décrite dans la demande FR1901084 déposée le 04/02/2019. Durant le fonctionnement, l'humidificateur 6 est disposé en dessous de la chambre de ventilation 5, ainsi que de l'alimentation électrique et les cartes électroniques de commande. En cas de fuite dans le réservoir d'eau, l'eau s'écoule par gravité sans atteindre la chambre de ventilation. Il en résulte une sécurité de fonctionnement accrue.

## Revendications

1. Dispositif de ventilation respiratoire (1), destiné à envoyer un flux d'air, généré par un ventilateur, dans un conduit (8), le conduit s'étendant entre le dispositif et un masque respiratoire (9), destiné à être porté par un utilisateur, le dispositif s'étendant entre une extrémité inférieure (3), formant un fond, et une extrémité supérieure (4), le dispositif comportant:
- une chambre de ventilation (5), renfermant le ventilateur ;
- un humidificateur (6), relié à la chambre de ventilation, et destiné à humidifier de l'air insufflé par le ventilateur, l'humidificateur comportant un réservoir (25) destiné à recevoir de l'eau ;
l'humidificateur comportant :
- une chambre de liaison (10), adjacente de la chambre de ventilation (5), et comprenant :
· une ouverture d'admission (13ᵢ), débouchant de la chambre de ventilation ;
· une ouverture d'évacuation (13ₒ), débouchant sur un canal d'évacuation (19), le canal d'évacuation étant configuré pour être raccordé au conduit (8);
- une chambre d'humidification (20), comportant le réservoir d'eau ;
- une première ouverture (15₁) et une deuxième ouverture (15₂), distinctes l'une de l'autre, formées entre la chambre de liaison (10) et la chambre d'humidification (20);
le dispositif étant **caractérisé en ce que** :
- la chambre de liaison (10) s'étend entre la chambre d'humidification (20) et la chambre de ventilation (5) ;
- le dispositif comporte une paroi de séparation (12) s'étendant entre la chambre de liaison (10) et la chambre d'humidification (20), la première ouverture (15₁) et la deuxième ouverture (15₂) étant ménagées dans la paroi de séparation, la paroi de séparation étant agencée de telle sorte que :
• la chambre d'humidification (20) s'étend entre le fond et la paroi de séparation (12);
• la chambre de liaison (10) s'étend entre la paroi de séparation (12) et la chambre de ventilation (5);
- la chambre de liaison définit un premier canal (16₁) entre l'ouverture d'admission et la première ouverture, ainsi qu'un deuxième canal (16₂) entre la deuxième ouverture et l'ouverture d'évacuation, de telle sorte que :
- le premier canal est agencé pour diriger de l'air, provenant la chambre de ventilation (5), à travers l'ouverture d'admission (13ᵢ), jusqu'à la chambre d'humidification, à travers la première ouverture (15₁);
- le deuxième canal est agencé pour diriger de l'air, provenant de la chambre d'humidification (20), à travers la deuxième ouverture (15₂), jusqu'à la l'ouverture d'évacuation (13ₒ).

2. Dispositif selon la revendication 1, comportant une paroi latérale annulaire (7), reliant l'extrémité inférieure (3) à l'extrémité supérieure (4), de telle sorte que la chambre de liaison (10), la chambre d'humidification (20) et la chambre de ventilation (5) sont délimitées par la paroi latérale annulaire.

3. Dispositif selon l'une quelconque des revendications précédentes, dans lequel le deuxième canal (16₂) définit un cheminement à travers la chambre de liaison (10), le cheminement comportant plusieurs coudes entre la deuxième ouverture (15₂) et l'ouverture d'évacuation (13ₒ).

4. Dispositif selon l'une quelconque des revendications précédentes, dans lequel :
- le premier canal (16₁) s'étend entre deux premières parois transversales (17₁), chaque première paroi transversale s'étendant entre la paroi de séparation (12) et une paroi supérieure (11) de la chambre de liaison ;
- et/ou le deuxième canal (16₂) s'étend entre deux deuxièmes parois transversales (17₂) chaque deuxième paroi transversale s'étendant entre la paroi de séparation (12) et la paroi supérieure (11) de la chambre de liaison.

5. Dispositif selon la revendication 4, dans lequel :
- la chambre de liaison (10) s'étend entre la paroi de séparation (12) et la paroi supérieure (11) de la chambre de liaison;
- chaque première paroi transversale (17₁) et/ou chaque deuxième paroi transversale (17₂) s'étend à partir de la paroi supérieure (11) de la chambre de liaison.

6. Dispositif selon l'une quelconque des revendications précédentes, comportant un obturateur, et dans lequel une ouverture, choisie parmi l'ouverture d'admission (13ᵢ) ou la première ouverture (15₁), est associée à l'obturateur (14), l'obturateur étant configuré pour passer d'une position ouverte, selon laquelle l'ouverture est ouverte, et une position fermée, selon laquelle l'ouverture est fermée, lorsque le dispositif s'incline, au-delà d'une inclinaison critique, par rapport à une position de référence du dispositif.

7. Dispositif selon la revendication 6, dans lequel l'obturateur (14) est apte à commuter, par gravité, entre la position ouverte et la position fermée.

8. Dispositif selon l'une quelconque des revendications précédentes, comportant un inclinomètre, configuré pour mesuré un angle d'inclinaison du dispositif par rapport à une position de référence du dispositif, l'inclinomètre étant relié à un coupe-circuit, l'inclinomètre étant configuré pour activer le coupe-circuit, de façon à interrompre un fonctionnement du ventilateur lorsque le dispositif s'incline, au-delà d'une inclinaison critique, par rapport à une position de référence du dispositif.

9. Dispositif selon l'une quelconque des revendications 2 à 8, dans lequel la paroi latérale annulaire (7) s'étend autour d'un axe transversal (Z), s'étendant entre le fond et l'extrémité supérieure.

10. Dispositif selon la revendication 9, dans laquelle la paroi annulaire (7) est cylindrique ou tronc-conique.

11. Dispositif selon l'une quelconque des revendications précédentes, dans lequel le réservoir d'eau (25) s'étend, dans la chambre d'humidification (20), entre le fond du dispositif (3) et la paroi de séparation (12).

12. Dispositif selon l'une quelconque des revendications précédentes, dans lequel le réservoir (25) d'eau est amovible, de telle sorte qu'il peut être extrait de la chambre d'humidification (20) ou inséré dans cette dernière.

13. Dispositif selon la revendication 12, dans lequel le réservoir d'eau (25) est mobile en translation par rapport au châssis, de façon à pouvoir être extrait ou inséré dans la chambre d'humidification par translation parallèlement au fond (3).

14. Dispositif selon l'une quelconque des revendications précédentes, dans lequel l'humidificateur (6) est amovible, de façon à pouvoir être séparé de la chambre de ventilation (5).

15. Dispositif selon l'une quelconque des revendications précédentes, dans lequel :
- la chambre de liaison est délimitée par une paroi supérieure (11), la paroi supérieure (11) étant adjacente de la chambre de ventilation (5) ;
- la paroi supérieure (11) est amovible.

## Patentansprüche

1. Beatmungsvorrichtung (1), die dazu bestimmt ist, einen durch einen Ventilator erzeugten Luftstrom in eine Leitung (8) zu leiten, wobei sich die Leitung zwischen der Vorrichtung und einer Atemmaske (9) erstreckt, die dazu bestimmt ist, von einem Benutzer getragen zu werden, wobei sich die Vorrichtung zwischen einem unteren Ende (3), das einen Boden bildet, und einem oberen Ende (4) erstreckt, wobei die Vorrichtung Folgendes aufweist:
- eine Ventilatorkammer (5), die den Ventilator umschließt;
- einen Luftbefeuchter (6), der mit der Ventilatorkammer verbunden und dazu bestimmt ist, die vom Ventilator eingeblasene Luft zu befeuchten, wobei der Luftbefeuchter einen Behälter (25) zur Aufnahme von Wasser aufweist; wobei der Luftbefeuchter Folgendes aufweist:
- eine Verbindungskammer (10), die an die Ventilatorkammer (5) angrenzt und Folgendes aufweist:
• eine Einlassöffnung (13i), die aus der Ventilatorkammer herausführt;
• eine Auslassöffnung (13ₒ), die in eine Auslassleitung (19) mündet, wobei die Auslassleitung dazu konfiguriert ist, mit der Leitung (8) verbunden zu sein;
- eine Befeuchtungskammer (20), die den Wasserbehälter enthält;
- eine erste Öffnung (15₁) und eine zweite Öffnung (15₂), die voneinander verschieden sind und zwischen der Verbindungskammer (10) und der Befeuchtungskammer (20) ausgebildet sind;
wobei die Vorrichtung **dadurch gekennzeichnet ist, dass**:
- sich die Verbindungskammer (10) zwischen der Befeuchtungskammer (20) und der Ventilatorkammer (5) erstreckt;
- die Vorrichtung eine Trennwand (12) aufweist, die sich zwischen der Verbindungskammer (10) und der Befeuchtungskammer (20) erstreckt, wobei die erste Öffnung (15₁) und die zweite Öffnung (15₂) in der Trennwand ausgebildet sind, wobei die Trennwand so angeordnet ist, dass:
• sich die Befeuchtungskammer (20) zwischen dem Boden und der Trennwand (12) erstreckt;
• sich die Verbindungskammer (10) zwischen der Trennwand (12) und der Ventilatorkammer (5) erstreckt;
- die Verbindungskammer einen ersten Kanal (16₁) zwischen der Einlassöffnung und der ersten Öffnung sowie einen zweiten Kanal (16₂) zwischen der zweiten Öffnung und der Auslassöffnung definiert, sodass:
- der erste Kanal so angeordnet ist, dass er Luft aus der Ventilatorkammer (5) durch die Einlassöffnung (13i) zur Befeuchtungskammer durch die erste Öffnung (15₁) leitet;
- der zweite Kanal so angeordnet ist, dass er Luft aus der Befeuchtungskammer (20) durch die zweite Öffnung (15₂) zur Auslassöffnung (13ₒ) leitet.

2. Vorrichtung nach Anspruch 1, die eine ringförmige Seitenwand (7) aufweist, die das untere Ende (3) mit dem oberen Ende (4) verbindet, so dass die Verbindungskammer (10), die Befeuchtungskammer (20) und die Ventilatorkammer (5) durch die ringförmige Seitenwand begrenzt sind.

3. Vorrichtung nach einem der vorangehenden Ansprüche, wobei der zweite Kanal (16₂) einen Weg durch die Verbindungskammer (10) definiert, wobei der Weg zwischen der zweiten Öffnung (15₂) und der Auslassöffnung (13ₒ) mehrere Biegungen aufweist.

4. Vorrichtung nach einem der vorangehenden Ansprüche, wobei:
- sich der erste Kanal (16₁) zwischen zwei ersten Querwänden (17₁) erstreckt, wobei sich jede erste Querwand zwischen der Trennwand (12) und einer oberen Wand (11) der Verbindungskammer erstreckt; und/oder
- sich der zweite Kanal (16₂) zwischen zwei zweiten Querwänden (17₂) erstreckt, wobei sich jede zweite Querwand zwischen der Trennwand (12) und der oberen Wand (11) der Verbindungskammer erstreckt.

5. Vorrichtung nach Anspruch 4, wobei:
- sich die Verbindungskammer (10) zwischen der Trennwand (12) und der oberen Wand (11) der Verbindungskammer erstreckt;
- sich jede erste Querwand (17₁) und/oder jede zweite Querwand (17₂) von der oberen Wand (11) der Verbindungskammer erstreckt.

6. Vorrichtung nach einem der vorangehenden Ansprüche, die einen Verschluss aufweist, wobei eine aus der Einlassöffnung (13i) und der ersten Öffnung (15₁) ausgewählte Öffnung mit dem Verschluss (14) verbunden ist, wobei der Verschluss so konfiguriert ist, dass er aus einer offenen Position, in der die Öffnung offen ist, in eine geschlossene Position, in der die Öffnung geschlossen ist, übergeht, wenn die Vorrichtung in Bezug auf eine Referenzposition der Vorrichtung über eine kritische Neigung hinaus geneigt wird.

7. Vorrichtung nach Anspruch 6, wobei der Verschluss (14) dazu geeignet ist, durch Schwerkraft zwischen der offenen Position und der geschlossenen Position zu wechseln.

8. Vorrichtung nach einem der vorangehenden Ansprüche, die einen Neigungsmesser aufweist, der dazu konfiguriert ist, einen Neigungswinkel der Vorrichtung in Bezug auf eine Referenzposition der Vorrichtung zu messen, wobei der Neigungsmesser mit einem Stromunterbrecher verbunden ist, wobei der Neigungsmesser dazu konfiguriert ist, den Stromunterbrecher zu aktivieren, um den Betrieb des Ventilators zu unterbrechen, wenn die Vorrichtung in Bezug auf eine Referenzposition der Vorrichtung über eine kritische Neigung hinaus geneigt wird.

9. Vorrichtung nach einem der Ansprüche 2 bis 8, wobei sich die ringförmige Seitenwand (7) um eine Querachse (Z) erstreckt, die sich zwischen dem Boden und dem oberen Ende erstreckt.

10. Vorrichtung nach Anspruch 9, wobei die ringförmige Wand (7) zylinder- oder kegelstumpfförmig ist.

11. Vorrichtung nach einem der vorangehenden Ansprüche, wobei sich der Wasserbehälter (25) in der Befeuchtungskammer (20), zwischen dem Boden der Vorrichtung (3) und der Trennwand (12) erstreckt.

12. Vorrichtung nach einem der vorangehenden Ansprüche, wobei der Wasserbehälter (25) abnehmbar ist, sodass er aus der Befeuchtungskammer (20) herausgenommen oder in diese eingesetzt werden kann.

13. Vorrichtung nach Anspruch 12, wobei der Wasserbehälter (25) in Bezug auf den Rahmen translatorisch bewegbar ist, sodass er durch Translation parallel zum Boden (3) aus der Befeuchtungskammer herausgenommen oder in diese eingesetzt werden kann.

14. Vorrichtung nach einem der vorangehenden Ansprüche, wobei der Luftbefeuchter (6) abnehmbar ist, sodass er von der Ventilatorkammer (5) getrennt werden kann.

15. Vorrichtung nach einem der vorangehenden Ansprüche, wobei:
- die Verbindungskammer durch eine obere Wand (11) begrenzt ist, wobei die obere Wand (11) an die Ventilatorkammer (5) angrenzt;
- die obere Wand (11) abnehmbar ist.

## Claims

1. A respiratory ventilation device (1), intended to send a flow of air, generated by a fan, into a duct (8), the duct extending between the device and a respiratory mask (9), intended to be worn by a user, the device extending between a lower end (3), forming a bottom, and an upper end (4), the device having:
- a ventilation chamber (5), containing the fan;
- a humidifier (6), connected to the ventilation chamber, and intended to humidify the air blown in by the fan, the humidifier having a reservoir (25) intended to accommodate water;
the humidifier having:
- a connecting chamber (10), adjacent to the ventilation chamber (5), and comprising:
• an intake opening (13i), leading out of the ventilation chamber;
• an exhaust opening (13ₒ), leading into an exhaust channel (19), the exhaust channel being configured to be attached to the duct (8);
- a humidification chamber (20), having the water reservoir;
- a first opening (15₁) and a second opening (15₂), separate from one another, formed between the connecting chamber (10) and the humidification chamber (20);
the device being **characterized in that**:
- the connecting chamber (10) extends between the humidification chamber (20) and the ventilation chamber (5);
- the device has a separating wall (12) extending between the connecting chamber (10) and the humidification chamber (20), the first opening (15₁) and the second opening (15₂) being made in the separating wall, the separating wall being arranged such that:
• the humidification chamber (20) extends between the bottom and the separating wall (12);
• the connecting chamber (10) extends between the separating wall (12) and the ventilation chamber (5).
- the connecting chamber defines a first channel (16₁) between the intake opening and the first opening, and also a second channel (16₂) between the second opening and the exhaust opening, such that:
- the first channel is arranged to direct air from the ventilation chamber (5) through the intake opening (13i) to the humidification chamber, through the first opening (15₁);
- the second channel is arranged to direct air from the humidification chamber (20) through the second opening (15₂) to the exhaust opening (13ₒ).

2. The device as claimed in claim 1, having an annular lateral wall (7), connecting the lower end (3) to the upper end (4), such that the connecting chamber (10), the humidification chamber (20) and the ventilation chamber (5) are delimited by the annular lateral wall.

3. The device as claimed in any one of the preceding claims, wherein the second channel (16₂) defines a path through the connecting chamber (10), the path having multiple bends between the second opening (15₂) and the exhaust opening (13ₒ).

4. The device as claimed in either one of claims 3 and 4, wherein:
- the first channel (16₁) extends between two first transverse walls (17₁), each first transverse wall extending between the separating wall (12) and an upper wall (11) of the connecting chamber;
- and/or the second channel (16₂) extends between two second transverse walls (17₂), each second transverse wall extending between the separating wall (12) and the upper wall (11) of the connecting chamber.

5. The device as claimed in claim 4, wherein:
- the connecting chamber (10) extends between the separating wall (12) and the upper wall (11) of the connecting chamber;
- each first transverse wall (17₁) and/or each second transverse wall (17₂) extends from the upper wall (11) of the connecting chamber.

6. The device as claimed in any one of the preceding claims, having a shutter, and wherein an opening, chosen from among the intake opening (13i) and the first opening (15₁), is associated with the shutter (14), the shutter being configured to pass from an open position, in which the opening is open, and a closed position, in which the opening is closed, when the device is tilted beyond a critical inclination with respect to a reference position of the device.

7. The device as claimed in claim 6, wherein the shutter (14) is able to switch between the open position and the closed position by gravity.

8. The device as claimed in any one of the preceding claims, having an inclinometer, configured to measure an angle of inclination of the device with respect to a reference position of the device, the inclinometer being connected to a circuit breaker, the inclinometer being configured to activate the circuit breaker so as to interrupt operation of the fan when the device is tilted beyond a critical inclination with respect to a reference position of the device.

9. The device as claimed in any one of claims 2 to 8, wherein the annular lateral wall (7) extends about a transverse axis (Z), extending between the bottom and the upper end.

10. The device as claimed in claim 9, wherein the annular wall (7) is cylindrical or frustoconical.

11. The device as claimed in any one of the preceding claims, wherein the water reservoir (25) extends, in the humidification chamber (20), between the bottom (3) of the device and the separating wall (12).

12. The device as claimed in any one of the preceding claims, wherein the water reservoir (25) is removable, such that it can be withdrawn from the humidification chamber (20) or inserted into the latter.

13. The device as claimed in claim 12, wherein the water reservoir (25) is movable in translation with respect to the frame, so as to be able to be withdrawn from or inserted into the humidification chamber by being moved in translation parallel to the bottom (3).

14. The device as claimed in any one of the preceding claims, wherein the humidifier (6) is removable so as to be able to be separated from the ventilation chamber (5).

15. The device as claimed in any one of the preceding claims, wherein:
- the connecting chamber is delimited by an upper wall (11), the upper wall (11) being adjacent to the ventilation chamber (5);
- the upper wall (11) is removable.
